# EUROPEAN PATENT SPECIFICATION

(11) **EP 2 595 684 B1**
(45) Date of publication and mention of the grant of the patent: **04.03.2015**
(21) Application number: 11743588.3
(22) Date of filing: 19.07.2011
(51) Int. Cl.: A61M 15/00

(54) **INHALER**
INHALATOR
INHALATEUR

(30) Priority: 21.07.2010 US 366305 P
(43) Date of publication of application: 29.05.2013
(73) Proprietor: AstraZeneca AB, 151 85 Södertälje (SE)
(72) Inventor: ARVIDSSON, Lars, Rune, Gustav, SE-212 14 Malmö (SE); BAKEWELL, William, Royston Hertfordshire SG8 6EE (GB); CAMPBELL, Patrick, Royston Hertfordshire SG8 6EE (GB); GROOMBRIDGE, Christopher Benjamin James, Royston Hertfordshire SG8 6EE (GB); JOHN, James, Daniel, Royston Hertfordshire SG8 6EE (GB); LUNDGREN, Jan, Olof Bertil, SE-212 14 Malmö (SE); LUNDSTRÖM, Camilla, SE-212 14 Malmö (SE); RITTFELDT, Mårten, SE-212 14 Malmö (SE); SVENNBERG, Jonas, SE-212 14 Malmö (SE)
(86) International application number: PCT/GB2011/051350
(87) International publication number: WO 2012/010878

(56) References cited:
- WO-A1-2010/042036
- US-B1- 6 328 034

## Description

### Technical field

The present invention relates to an inhaler having a mouthpiece (or nosepiece) and a cover which is movable so as either to enclose the mouthpiece to protect it when the inhaler is stored, or to or expose the mouthpiece for use.

### Background of the Invention

There are different types of inhalers on the market. A pressurized metered dose inhaler (pMDI) releases a fixed dose of substance in aerosol form. A powder inhaler generally releases a dose of powdered substance entrained in an air stream. In a powder inhaler the powder may be provided in a bulk container of the inhaler from which doses of powder are metered for dispensing. As an alternative to a bulk container, powder inhalers may comprise a single compartment or a plurality of compartments for containing one or more discrete doses of powdered substance. Such compartments may take the form of sealed blisters in a blister pack, a flexible strip of sealed cavities or other suitable forms.

Whatever the type of inhaler, an air inlet is normally required through which air is drawn into and then through the inhaler when the user inhales through the mouthpiece (or nose piece in some cases). In a dry powder inhaler, the air flow path is carefully designed so that the air passing through the inhaler entrains and deaggregates dry powder medicament, but pressurised metered dose inhalers also need to allow a flow of air to be taken into the lungs of a user at the same time as the medicament, and this would normally be drawn through the inhaler and then out of the mouthpiece together with the medicament.

A problem can arise if the user inadvertently covers the inlet with his or her hand while using the inhaler. The flow of air to the user may be reduced and this may affect the delivered dose of medicament without it being obvious to the user that anything is amiss. It is therefore desirable for the air inlet in an inhaler to be positioned in such a way that makes it difficult for a user to obstruct it when using the inhaler.

It is desirable for the air inlet as well as the mouthpiece to be covered when the inhaler is not in use to avoid contamination of the airway with foreign substances (e.g. dust from a pocket in which the inhaler is carried). Inadvertent inhalation of such substances is to be avoided.

It is desirable for an inhaler to have a mouthpiece which has sufficient length to allow the user to make a good seal around it with their mouth. The inhaler is preferably also aesthetically pleasing for the user, which can sometimes be at odds with the objective of having a long mouthpiece.

It is desirable to avoid creating additional air flow paths from the atmosphere into a main flow path from the air inlet to the mouthpiece. One or more bypass air flow channels may be provided whose flow characteristics are carefully defined and whose contribution to the overall pressure drop and flow rate when the inhaler is used is carefully factored in. However, so-called leakage flow through e.g. ill-fitting components, apertures created as part of a moulding process, windows in the inhaler casing for displaying a dose count, etc, is desirably to be avoided.

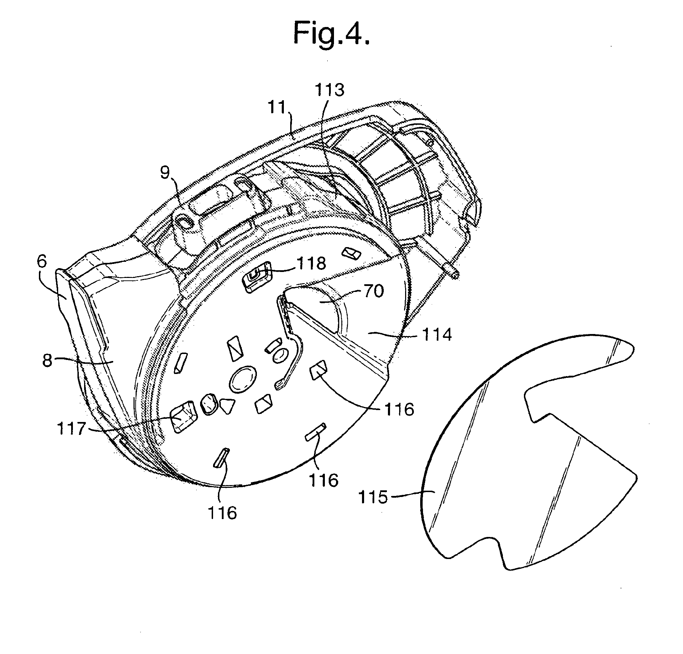

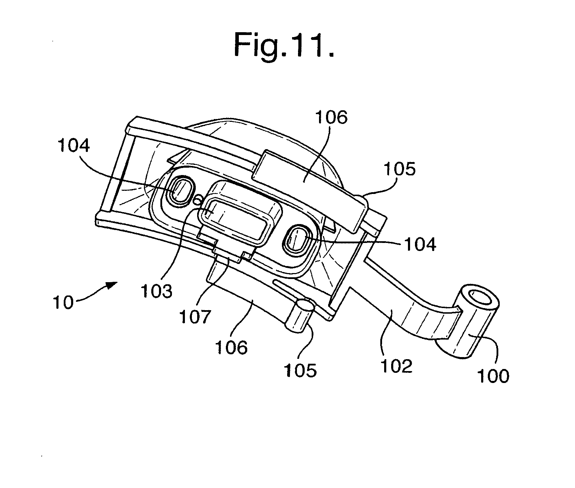

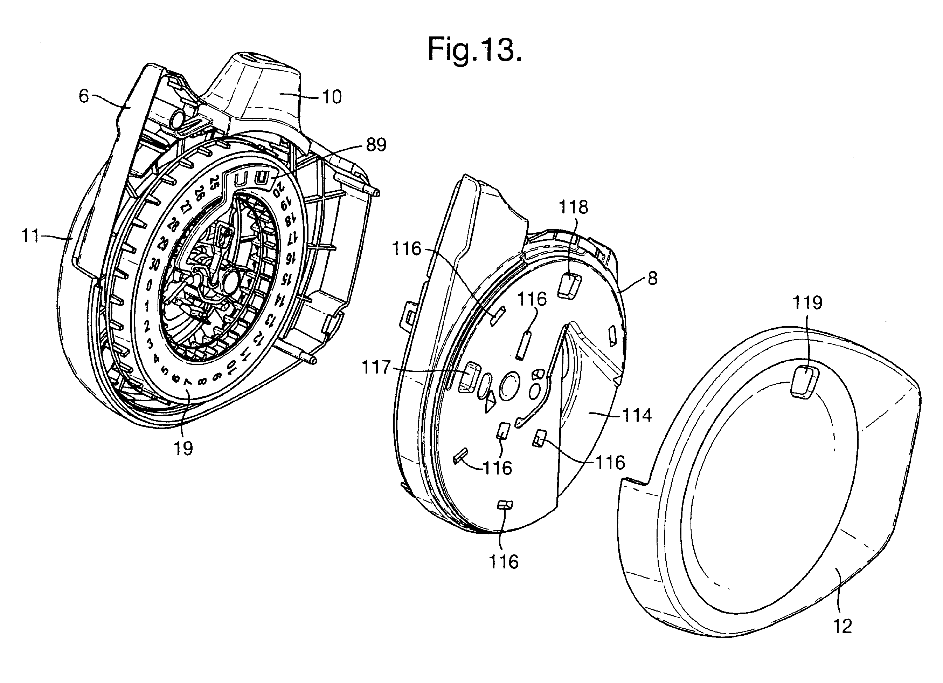

One design of dry powder inhaler is disclosed in US5590645 (Glaxo). This dry powder inhaler has the general form of a disc, with the mouthpiece provided at the edge of the disc. A cover is pivoted to the central axis of the disc; the cover being generally flush with the overall profile of the device to make for a device with a generally pleasing overall appearance. The air inlet is located in one of the major faces of the disc; this is clearly the case in the product corresponding to this patent, known as Diskus® (trade mark of GlaxoSmithKline plc). In US5590645 Figures 13-16 show the closest embodiment to the marketed product, though the location of the air inlet(s) is not clear; however, the location of the inlet can be deduced from the air flow diagram in Figures 4a and 11. It may be possible for a user to block or partially block this inlet when holding the inhaler in order to use it. The mouthpiece in this inhaler (referring the commercial product and to Figures 13-16) is both relatively short and also recessed with respect to the edge profile of the inhaler; this allows the cover to be substantially flush with the overall profile of the device.

US 6 328 034 discloses a dry powder inhaler according to the preamble of claim 1.

### Summary of the Invention

An object of the present invention is to avoid drawbacks associated with some prior inhalers. This and other objects, which will become apparent in the following, are accomplished by the inhaler defined in the accompanying claims.

An inhaler comprises:
- a housing including a mouthpiece;
- a cover movably mounted to the housing so as to be movable between an open configuration of the inhaler in which the mouthpiece is exposed for use and a closed configuration of the inhaler in which the mouthpiece is enclosed;
- wherein, in the open position, the cover at least partly defines an inlet aperture to an inhalation air flow path leading through the inhaler and communicating with the mouthpiece and wherein, in the closed position, the mouthpiece is received into the inlet aperture.

In one embodiment, the inlet aperture may be defined between the housing and the cover when the cover is in the open configuration. In the closed configuration, the inlet aperture may be closed. In the open configuration, the inlet aperture may be located adjacent the mouthpiece.

The inhaler has an overall flat shape, with opposed major external surfaces; the mouthpiece may, in the open configuration, project from between the said major surfaces. The air inlet aperture, in the open configuration, may also be located between the major surfaces.

The major surfaces are, at least in the closed configuration, provided substantially entirely by the cover, for example at least 90%, preferably at least 95% of the major surfaces may be provided by the cover.

The housing may have an overall disc shape, the mouthpiece projecting from an edge of the disc and extending beyond the overall disc profile. The housing may also have a further portion projecting from the disc and extending beyond the overall disc profile which may function, among other things, as a handle for a user to move the housing with respect to the cover thereby moving between open and closed configurations of the inhaler. The cover may be pivotally mounted on the housing.

Inside the housing may be located e.g. a dry powder reservoir or series of cavities filled with dry powder or a blister strip, together with various mechanisms for metering powder for an inhalation or for opening a cavity or blister or indexing a strip or disc of powder cavities, etc. Various formations may be required to mount these mechanisms and these are preferably integrally moulded with one or other half of a two-part moulding making up the housing. The manufacture of the housing with such mountings (e.g. snap fitting projections, etc) may involve one or more holes being created in the housing during the moulding process. These are both technically undesirable since they may create undesirable leakage paths in the air flow passage or passages through the inhaler, and also could detract from the aesthetics of the inhaler if they were not covered.

The cover may extend over any such moulding holes created in the housing, concealing them from view. A film, which may be self adhesive, may be applied to one or both major surfaces of the housing in order to seal the holes and prevent leakage of air into the flow path via the holes.

It may be desirable for a display to be provided e.g. indicating the number of the dose or number of remaining doses, or indicating whether a dose is ready to be inhaled, or some other indication. Such a display would normally be viewable through a viewing aperture in a housing which houses the mechanism associated with the provision of doses of medicament for inhalation. It is normally desirable to provide a transparent cover over any such viewing aperture to avoid contamination of the inhaler and to avoid air leakage; this would conventionally take the form of a separate moulding of transparent plastics material. If the housing has a film applied to it as described above, this film could extend over any display aperture and be transparent, thereby sealing the display aperture and allowing the display to be viewed. If the cover extends over the display aperture or apertures, then it may be provided with an outer display aperture or apertures through which the display or displays may be visible to a user.

Prior US patent application number 61/022854 (from which WO 2009/093969 claims priority) discusses the provision of an alternating display in an inhaler. In the closed configuration, a dose count display is visible; in the open configuration, the mouthpiece cover is moved over the dose count display but exposes a display indicating readiness (or not) of a dose for inhalation. When the cover is closed again, the dose count display is exposed and the readiness display is again concealed. This feature may be incorporated into an embodiment of the present invention, although realised in a different way. The housing may have a dose counter display window and a readiness display window. The cover may be provided with a secondary display window which, in the open configuration, is in registry with the readiness display window of the housing and, in the closed configuration, is in registry with the dose counter display window of the housing.

In one example, an inhaler comprises a mouthpiece and a housing in which is located a medicament dry powder reservoir or a series of cavities or blisters holding medicament dry powder, the housing also incorporating an air inlet and at least one display window (aperture) for displaying one or more indicia located within the housing, wherein a seal membrane is bonded to the housing, preferably to an outer wall of the housing, which seal membrane extends over the display window, wherein at least the part of the seal membrane which is in registry with the window is transparent.

The seal membrane is preferably made from a suitable polymer such as polyethylene and is preferably self adhesive, that is to say it is manufactured separately with an adhesive layer so that assembly of the seal to the inhaler involves simply applying the self adhesive seal membrane to the housing

The housing may have one or more further apertures in it, e.g. apertures connected with the moulding of the housing and which have no function in the finished inhaler, which are sealed by the seal membrane.

The inhaler may also comprise an external cover mounted on the housing which may be movable between an open configuration of the inhaler in which the mouthpiece is exposed for use and a closed configuration of the inhaler in which the mouthpiece is enclosed. The cover may have one or more outer display windows located so as to come into registry with one or more of the said at least one display window in the housing, when the inhaler is in either the closed or open configuration. An outer display window may be located so as to come into registry with different display windows in the housing depending on whether the inhaler is in an open or closed configuration.

The medicament of the medicament dry powder in the inhaler may contain various active ingredients. The active ingredient may be selected from any therapeutic or diagnostic agent. For example, the active ingredient may be an antiallergic, a bronchodilator (e.g. a beta2-adrenoceptor agonist or a muscarinic antagonist), a bronchoconstrictor, a pulmonary lung surfactant, an analgesic, an antibiotic, a mast cell inhibitor, an antihistamine, an anti-inflammatory, an antineoplastic, an anaesthetic, an antitubercular, an imaging agent, a cardiovascular agent, an enzyme, a steroid, genetic material, a viral vector, an antisense agent, a protein, a peptide, a non-steroidal glucocorticoid Receptor (GR Receptor) agonist, an antioxidant, a chemokine antagonist (e.g. a CCR1 antagonist), a corticosteroid, a CRTh2 antagonist, a DP1 antagonist, an Histone Deacetylase Inducer, an IKK2 inhibitor, a COX inhibitor, a lipoxygenase inhibitor, a leukotriene receptor antagonist, an MPO inhibitor, a p38 inhibitor, a PDE inhibitor, a PPARγ agonist, a protease inhibitor, a statin, a thromboxane antagonist, a vasodilator, an ENAC blocker (Epithelial Sodium-channel blocker) and combinations thereof.

Examples of specific active ingredients that can be incorporated in the inhaler include:
(i) antioxidants:- Allopurinol, Erdosteine, Mannitol, N-acetyl cysteine choline ester, N-acetyl cysteine ethyl ester, N-Acetylcysteine, N-Acetylcysteine amide and Niacin;
(ii) chemokine antagonists:- BX471 ((2R)-1-[[2-[(aminocarbonyl)amino]-4-chlorophenoxy]acetyl]-4-[(4-fluorophenyl)methyl]-2-methylpiperazine monohydrochloride), CCX634, *N*-{2-[((2*S*)-3-{[1-(4-chlorobenzyl)piperidin-4-yl]amino}-2-hydroxy-2-methylpropyl)oxy]-4-hydroxyphenyl}acetamide (see WO 2003/051839), and 2-{2-Chloro-5-{[(2S)-3-(5-chloro-1'H,3H-spiro[1-benzofuran-2,4'-piperidin]-1'-yl)-2-hydroxypropyl]oxy}-4-[(methylamino)carbonyl]phenoxy}-2-methylpropanoic acid (see WO 2008/010765), 656933 (N-(2-bromophenyl)-N'-(4-cyano-1H-1,2,3-benzotriazol-7-yl)urea), 766994 (4-({[({[(2R)-4-(3,4-dichlorobenzyl)morpholin-2-yl]methyl}amino)carbonyl]-amino}methyl)benzamide), CCX-282, CCX-915, Cyanovirin N, E-921, INCB-003284, INCB-9471, Maraviroc, MLN-3701, MLN-3897, T-487 (N-{1-[3-(4-ethoxyphenyl)-4-oxo-3,4-dihydropyrido[2,3-d]pyrimidin-2-yl]ethyl}-N-(pyridin-3-ylmethyl)-2-[4-(trifluoromethoxy)phenyl]acetamide) and Vicriviroc
(iii) Corticosteroids: -Alclometasone dipropionate, Amelometasone, Beclomethasone dipropionate, Budesonide, Butixocort propionate, Ciclesonide, Clobetasol propionate, Desisobutyrylciclesonide, Etiprednol dicloacetate, Fluocinolone acetonide, Fluticasone Furoate, Fluticasone propionate, Loteprednol etabonate (topical) and Mometasone furoate.
(iv) DP1 antagonists:- L888839 and MK0525;
(v) Histone deacetylase inducers:- ADC4022, Aminophylline, a Methylxanthine or Theophylline;
(vi) IKK2 inhibitors:- 2-{[2-(2-Methylamino-pyrimidin-4-yl)-1H-indole-5-carbonyl]-amino}-3-(phenyl-pyridin-2-yl-amino)-propionic acid;
(vii) COX inhibitors:- Celecoxib, Diclofenac sodium, Etodolac, Ibuprofen, Indomethacin, Meloxicam, Nimesulide, OC1768, OC2125, OC2184, OC499, OCD9101, Parecoxib sodium, Piceatannol, Piroxicam, Rofecoxib and Valdecoxib;
(viii) Lipoxygenase inhibitors:- Ajulemic acid, Darbufelone, Darbufelone mesilate, Dexibuprofen lysine (monohydrate), Etalocib sodium, Licofelone, Linazolast, Lonapalene, Masoprocol, MN-001 , Tepoxalin, UCB-35440, Veliflapon, ZD-2138, ZD-4007 and Zileuton ((±)-1-(1-Benzo[b]thien-2-ylethyl)-1-hydroxyurea);
(ix) Leukotriene receptor antagonists:- Ablukast, Iralukast (CGP 45715A), Montelukast, Montelukast sodium, Ontazolast, Pranlukast, Pranlukast hydrate (mono Na salt), Verlukast (MK-679) and Zafirlukast;
(x) MPO Inhibitors:- Hydroxamic acid derivative (N-(4-chloro-2-methyl-phenyl)-4-phenyl-4-[[(4-propan-2-ylphenyl)sulfonylamino]methyl]piperidine-1-carboxamide), Piceatannol and Resveratrol;
(xi) Beta2-adrenoceptor agonists:- metaproterenol, isoproterenol, isoprenaline, albuterol, salbutamol (e.g. as sulphate), formoterol (e.g. as fumarate), salmeterol (e.g. as xinafoate), terbutaline, orciprenaline, bitolterol (e.g. as mesylate), pirbuterol, indacaterol, salmeterol (e.g. as xinafoate), bambuterol (e.g. as hydrochloride), carmoterol, indacaterol (CAS no 312753-06-3; QAB-149), formanilide derivatives e.g. 3-(4-{[6-({(2R)-2-[3-(formylamino)-4-hydroxyphenyl]-2-hydroxyethyl}amino)hexyl]oxy}-butyl)-benzenesulfonamide; 3-(4-{[6-({(2R)-2-hydroxy-2-[4-hydroxy-3-(hydroxy-methyl)phenyl]ethyl}amino)-hexyl]oxy}butyl)benzenesulfonamide; GSK 159797, GSK 159802, GSK 597901, GSK 642444, GSK 678007; and a compound selected from *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide, *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(3-chlorophenyl)ethoxy]propanamide, 7-[(1*R*)-2-({2-[(3-{[2-(2-Chlorophenyl)ethyl]amino}propyl)thio]ethyl}amino)-1-hydroxyethyl]-4-hydroxy-1,3-benzothiazol-2(3*H*)-one, and *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. wherein the counter ion is hydrochloride (for example a monohydrochloride or a dihydrochloride), hydrobromide (for example a monohydrobromide or a dihydrobromide), fumarate, methanesulphonate, ethanesulphonate, benzenesulphonate, 2,5-dichlorobenzenesulphonate, *p-*toluenesulphonate, napadisylate (naphthalene-1,5-disulfonate or naphthalene-1-(sulfonic acid)-5-sulfonate), edisylate (ethane-1,2-disulfonate or ethane-1-(sulfonic acid)-2-sulfonate), D-mandelate, L-mandelate, cinnamate or benzoate.)
(xii) Muscarinic antagonists:- Aclidinium bromide, Glycopyrrolate (such as R,R-, R,S-, S,R-, or S,S-glycopyrronium bromide), Oxitropium bromide, Pirenzepine, telenzepine, Tiotropium bromide, 3(R)-1-phenethyl-3-(9H-xanthene-9-carbonyloxy)-1-azoniabicyclo[2.2.2]octane bromide, (3R)-3-[(2S)-2-cyclopentyl-2-hydroxy-2-thien-2-ylacetoxy]-1-(2-phenoxyethyl)-1-azoniabicyclo[2.2.2]actane bromide, a quaternary salt (such as [2-((R)-Cyclohexyl-hydroxy-phenyl-methyl)-oxazol-5-ylmethyl]-dimethyl-(3-phenoxy-propyl)-ammonium salt, [2-(4-Chloro-benzyloxy)-ethyl]-[2-((R)-cyclohexyl-hydroxy-phenyl-methyl)-oxazol-5-ylmethyl]- dimethyl-ammonium salt and (*R*)-1-[2-(4-Fluoro-phenyl)-ethyl]-3-((*S*)-2-phenyl-2-piperidin-1-yl-propionyloxy)-1-azonia-bicyclo[2.2.2]octane salt wherein the counter-ion is, for example, chloride, bromide, sulfate, methanesulfonate, benzenesulfonate (besylate), toluenesulfonate (tosylate), napthalenebissulfonate (napadisylate or hemi-napadisylate), phosphate, acetate, citrate, lactate, tartrate, mesylate, maleate, fumarate or succinate)
(xiii) p38 Inhibitors:- 681323, 856553, AMG548 (2-[[(2S)-2-amino-3-phenylpropyl]amino]-3-methyl-5-(2-naphthalenyl)-6-(4-pyridinyl)-4(3H)-pyrimidinone), Array-797, AZD6703, Doramapimod, KC-706, PH 797804, R1503, SC-80036, SCIO469, 6-chloro-5-[[(2*S*,5*R*)-4-[(4-fluorophenyl)methyl]-2,5-domethyl-1-piperazinyl]carbonyl]-*N*,*N*,1-trimethyl-α-oxo-1*H*-indole-3-acetamide, VX702 and VX745 (5-(2,6-dichlorophenyl)-2-(phenylthio)-6H-pyrimido[1,6-b]pyridazin-6-one);
(xiv) PDE Inhibitors:- 256066, Arofylline (3-(4-chlorophenyl)-3,7-dihydro-1-propyl-1H-Purine-2,6-dione), AWD 12-281 (N-(3,5-dichloro-4-pyridinyl)-1-[(4-fluorophenyl)methyl]-5-hydroxy-α-oxo-1H-indole-3-acetamide), BAY19-8004 (Bayer), CDC-801 (Calgene), Celgene compound ((βR)-β-(3,4-dimethoxyphenyl)-1,3-dihydro-1-oxo-2H-isoindole-2-propanamide), Cilomilast (cis-4-cyano-4-[3-(cyclopentyloxy)-4-methoxyphenyl]-cyclohexanecarboxylic acid), 2-(3,5-dichloro-4-pyridinyl)-1-(7-methoxyspiro[1,3-benzodioxole-2,1'-cyclopentan]-4-yl)ethanone (CAS number 185406-34-2)), (2-(3,4-difluorophenoxy)-5-fluoro-N-[cis-4-[(2-hydroxy-5-methylbenzoyl)amino]cyclohexyl]-)-3-pyridinecarboxamide), (2-(3,4-difluorophenoxy)-5-fluoro-N-[cis-4-[[2-hydroxy-5-(hydroxymethyl)benzoyl]amino]cyclohexyl]-3-pyridinecarboxamide,), CT2820, GPD-1116, Ibudilast, IC 485, KF 31334, KW-4490, Lirimilast ([2-(2,4-dichlorobenzoyl)-6-[(methylsulfonyl)oxy]-3-benzofuranyl])-urea), (N-cyclopropyl-1,4-dihydro-4-oxo-1-[3-(3-pyridinylethynyl)phenyl]-)-1,8-naphthyridine-3-carboxamide), (N-(3,5-dichloro-4-pyridinyl)-4-(difluoromethoxy)-8-[(methylsulfonyl)amino])-1-dibenzofurancarboxamide), ONO6126, ORG 20241 (4-(3,4-dimethoxyphenyl)-N-hydroxy-)-2-thiazolecarboximidamide), PD189659/PD168787 (Parke-Davis), Pentoxifylline (3,7-dihydro-3,7-dimethyl-1-(5-oxohexyl)-)-1H-purine-2,6-dione), compound (5-fluoro-N-[4-[(2-hydroxy-4-methyl-benzoyl)amino]cyclohexyl]-2-(thian-4-yloxy)pyridine-3-carboxamide), Piclamilast (3-(cyclopentyloxy)-N-(3,5-dichloro-4-pyridinyl)-4-methoxy-benzamide), PLX-369 (WO 2006026754), Roflumilast (3-(cyclopropylmethoxy)-N-(3,5-dichloro-4-pyridinyl)-4-(difluoromethoxy)benzamide), SCH 351591 (N-(3,5-dichloro-1-oxido-4-pyridinyl)-8-methoxy-2-(trifluoromethyl)-5-quinolinecarboxamide), SelClD(TM) CC-10004 (Calgene), T-440 (Tanabe), Tetomilast (6-[2-(3,4-diethoxyphenyl)-4-thiazolyl]-2-pyridinecarboxylic acid), Tofimilast (9-cyclopentyl-7-ethyl-6,9-dihydro-3-(2-thienyl)-5H-pyrazolo[3,4-c]-1,2,4-triazolo[4,3-a]pyridine), TPI 1100, UCB 101333-3 (N,2-dicyclopropyl-6-(hexahydro-1H-azepin-1-yl)-5-methyl-4-pyrimidinamine), V-11294A (Napp), VM554/VM565 (Vernalis), and Zardaverine (6-[4-(difluoromethoxy)-3-methoxyphenyl]-3(2H)-pyridazinone).
(xv) PDE5 Inhibitors:- Gamma-glutamyl[s-(2-iodobenzyl)cysteinyl]glycine, Tadalafil, Vardenafil, sildenafil, 4-phenyl-methylamino-6-chloro-2-(1-imidazolyl)-quinazoline, 4-phenyl-methylamino-6-chloro-2-(3-pyridyl)-quinazoline, 1,3-dimethyl-6-(2-propoxy-5-methanesulphonylamidophenyl)-1,5-dihydropyrazolo[3,4-d]pyrimidin-4-one and 1-cyclopentyl-3-ethyl-6-(3-ethoxy-4-pyridyl)-pyrazolo[3,4-d]pyrimidin-4-one;
(xvi) PPARγ agonists:- Pioglitazone, Pioglitazone hydrochloride, Rosiglitazone Maleate, Rosiglitazone Maleate ((-)-enantiomer, free base), Rosiglitazone maleate/Metformin hydrochloride and Tesaglitizar;
(xvii) Protease Inhibitors:- Alpha1-antitrypsin proteinase Inhibitor, EPI-HNE4, UT-77, ZD-0892, DPC-333, Sch-709156 and Doxycycline;
(xviii) Statins:- Atorvastatin, Lovastatin, Pravastatin, Rosuvastatin and Simvastatin
(xix) Thromboxane Antagonists: Ramatroban and Seratrodast;
(xx) Vasodilators:- A-306552, Ambrisentan, Avosentan, BMS-248360, BMS-346567, BMS-465149, BMS-509701, Bosentan, BSF-302146 (Ambrisentan), Calcitonin Gene-related Peptide, Daglutril, Darusentan, Fandosentan potassium, Fasudil, Iloprost, KC-12615 (Daglutril), KC-12792 2AB (Daglutril), Liposomal treprostinil, PS-433540, Sitaxsentan sodium, Sodium Ferulate, TBC-11241 (Sitaxsentan), TBC-3214 (N-(2-acetyl-4,6-dimethylphenyl)-3-[[(4-chloro-3-methyl-5-isoxazolyl)amino]sulfonyl]-2-thiophenecarboxamide), TBC-3711, Trapidil, Treprostinil diethanolamine and Treprostinil sodium;
(xxi) ENACs:- Amiloride, Benzamil, Triamterene, 552-02, PSA14984, PSA25569, PSA23682 and AER002.

The inhaler may contain a combination of two or more active ingredients, for example a combination of two or more of the specific active ingredients listed in (i) to (xxi) herein above.

In one embodiment the inhaler contains an active ingredient selected from mometasone, ipratropium bromide, tiotropium and salts thereof, salemeterol, fluticasone propionate, beclomethasone dipropionate, reproterol, clenbuterol, rofleponide and salts, nedocromil, sodium cromoglycate, flunisolide, budesonide, formoterol fumarate dihydrate, terbutaline, terbutaline sulphate, salbutamol base and sulphate, fenoterol, 3-[2-(4-Hydroxy-2-oxo-3H-1,3-benzothiazol-7-yl)ethylamino]-N-[2-[2-(4-methylphenyl)ethoxy]ethyl]propane-sulphonamide, hydrochloride, indacaterol, aclidinium bromide, *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide); *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. diD-mandelate); a [2-(4-Chloro-benzyloxy)-ethyl]-[2-((R)-cyclohexyl-hydroxy-phenylmethyl)-oxazol-5-ylmethyl]- dimethyl-ammonium salt (e.g. hemi-naphthalene-1,5-disulfonate); a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate); or a combination of any two or more thereof.

Specific combinations of active ingredients which may be incorporated in the inhaler include:-
(a) formoterol (e.g. as fumarate) and budesonide;
(b) formoterol (e.g. as fumarate) and fluticasone;
(c) *N*-[2-(Diethylamino)ethyl]-*N*-(2-{[2-(4-hydroxy-2-oxo-2,3-dihydro-1,3-benzothiazol-7-yl)ethyl] amino }ethyl)-3-[2-(1-naphthyl)ethoxy]propanamide or a pharmaceutically acceptable salt thereof (e.g. dihydrobromide) and a [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
(d) *N*-[2-(Diethylamino)ethyl]-*N*-(2-{[2-(4-hydroxy-2-oxo-2,3-dihydro-1,3-benzothiazol-7-yl)ethyl]amino}ethyl)-3-[2-(1-naphthyl)ethoxy]propanamide or a pharmaceutically acceptable salt thereof (e.g. dihydrobromide) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate);
(e) *N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. di-D-mandelate) and [2-(4-Chloro-benzyloxy)-ethyl]-[2-((R)-cyclohexyl-hydroxy-phenyl-methyl)-oxazol-5-ylmethyl]-dimethyl-ammonium salt (e.g. hemi-naphthalene-1,5-disulfonate);
*N*-Cyclohexyl-*N*³-[2-(3-fluorophenyl)ethyl]-*N*-(2-{[2-(4-hydroxy-2-oxo-2,3-dihydro-1,3-benzothiazol-7-yl)ethyl]amino}ethyl)-β-alaninamide or a pharmaceutically acceptable salt thereof (e.g. di-D-mandelate) and a (*R*)-1-[2-(4-Fluoro-phenyl)-ethyl]-3-((*S*)-2-phenyl-2-piperidin-1-yl-propionyloxy)-1-azonia-bicyclo[2.2.2]octane salt (e.g. bromide or toluenesulfonate).

### Definitions

Flat shall mean having a maximum extent in a first dimension less than 40% of the maximum extent in each of second and third dimensions, the three said dimensions being mutually perpendicular. Preferably, the first dimension is less than 30%, more preferably less than 25% of the maximum extent in each of the second and third dimensions.

Major face, in the context of an article described as having a flat shape as defined above, shall mean one of the two general surfaces defining the extent of the article in the second and third dimensions referred to above.

### Brief description of the drawings

Fig. 1 is a perspective view from above of an inhaler according to at least one example embodiment of the invention, in an open configuration;
Fig. 2 is a perspective view from above of the inhaler of Figure 1, in a closed configuration;
Fig. 3 is a perspective view from below of the inhaler of Figure 1, in an open configuration;
Fig. 4 is a perspective view from below of the inhaler of Figure 1, with an outer casing component removed;
Fig. 5 is an exploded perspective view of the inhaler of Figure 1;
Fig. 6 is a cross sectional view of selected components of the inhaler of Figure 1, in a primed condition;
Fig. 7 is a cross sectional view of selected components of the inhaler of Figure 1, in a fired condition;
Fig. 8 is a perspective view from below of a mouthpiece and upper outer casing component of the inhaler of Figure 1;
Fig. 9. is a plan view from below of the cavity disc and indexing mechanism of the inhaler of Figure 1;
Fig. 10 is a perspective view of part of the indexing mechanism and actuator of the inhaler of Figure 1;
Fig. 11 is a perspective view of the mouthpiece of the inhaler of Figure 1;
Fig. 12 is a plan view from above of the lower housing, drive member (indexer) and torsion spring; and
Fig. 13 is a perspective view of the inhaler of Figure 1 with the lower housing and lower cover separated, and with some components omitted for clarity.

### Detailed description of the drawings

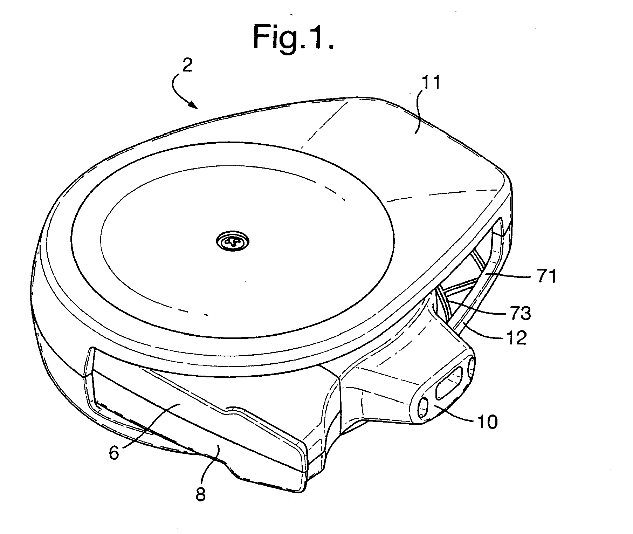

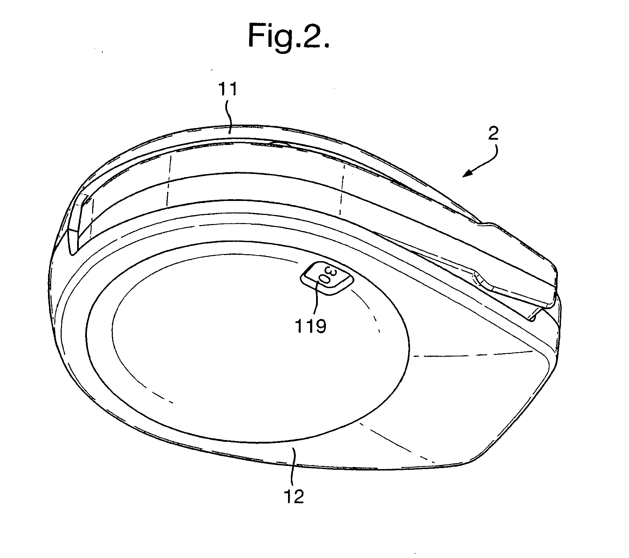

Referring to the Figures, the inhaler 2 comprises a dose dispensing assembly 4 having a general disc configuration, an upper housing portion 6 and a lower housing portion 8, both of 30% glass fibre reinforcement plastic (eg. polybutylene terephtalate, PBT). The inhaler also comprises a mouthpiece assembly, the assembly comprising a mouthpiece member 10 of polypropylene (e.g. Purell HM671T) with a thermoplastic vulcanizated elastomeric mouthpiece seal member 9 which seals the interface between the mouthpiece assembly and the housing. Alternatively, the seal member 9 could for example comprise an elastomeric member such as Santoprene 8281-45MED. The housing is pivotally mounted within a casing comprising upper and lower outer casing components 11, 12 of polycarbonate (e.g. Makrolon 2458); in this way the inhaler can be moved between open and closed configurations. In the open configuration the mouthpiece is exposed and an external air inlet 71 is opened up adjacent the mouthpiece (see e.g. Figure 1). In the closed configuration, the mouthpiece is enclosed within the outer cover 11, 12 and the air inlet 71 is shut (see e.g. Figure 2).

The dose dispensing assembly 4 comprises a cavity disc 14 of high density polyethylene (such as Purell GC7260) which has a plurality of cavities 16 formed in one major face of the disc, evenly spaced around the periphery. An alternative material for the dose dispensing assembly 4 is polypropylene (e.g. Purell HM671T). The cavities 16 contain dry powder medicament for inhalation (not shown), and are sealed by a laminated film 18 of aluminium foil and polymer material (referred to as a "foil layer"), thus providing sealed compartments. Above each cavity 16, a respective associated separating element 20 of polypropylene is attached to the upper side of the foil layer 18. Alternatively, the separating element can be formed of high density polyethylene (such as Purell GC7260) particularly when the dose dispensing assembly 4 is polypropylene (e.g. Purell HM671T). The separating elements 20 are attached by any suitable type of bonding, welding, gluing, etc. to the respective part of the foil layer 18. Upwards movement or lifting of a separating element 20 causes the attached part of the foil layer 18 to become separated from the cavity 16. The foil layer 18 has radial cuts between each separating element.

On the opposite face of the cavity disc 14 is a second annular foil layer 19 (see Figure 13) bearing numbers 1-30 corresponding to the medicament cavities 16. Underneath the cavities a space is defined extending around the disc. This space contains a desiccant material(either as molecular sieve or silica gel), and the second foil layer 19 seals the desiccant in this space. In certain states of the inhaler, one of the numbers printed on the second foil are visible though windows in the lower housing portion 8 and lower cover 12. This is explained more fully below.

A circular guide structure 22 of of high density polyethylene is provided above the separating elements 20. The guide structure could alternatively be formed of polypropylene (e.g. Purell HM671T). The guide structure 22 comprises a plurality of guide sections 24 divided by vertically extending walls, each guide section 24 being associated with a respective separating element 20. When a separating element 20 is lifted from the cavity disc 14, the associated guide section 24 will guide the upwards movement of the separating element 20. Each guide section 24 is provided with a blade spring 26 as part of the moulding. The blade spring bears downwardly on the top of the respective separating element 20. After a separating element 20 has been lifted and medicament in the opened cavity 16 has been entrained in the inhalation airflow and the separating element 20 has returned to the disc 14, the blade spring 26 will keep the lifted separating element 20 in contact with the disc 14 to cover the cavity 16. This will make it difficult for any remaining powder to exit the covered used cavity 16, thus reducing the risk of dose variation which could occur if remaining powder were to be entrained in a following inhalation. It also reduces the risk of remaining powder exiting the cavity 16 and jamming mechanical components in the inhaler or the risk of the separating element creating a rattling noise which would be undesirable for the user.

The vertical walls dividing the circular guide structure 22 into guide sections 24 function as lateral flow path defining elements. Thus, an inhalation airflow is prevented from deviating sideways once it reaches the cavity area of the disc 14 and will be led to the mouthpiece 10. An alternative would be to have shorter vertical walls, in which case neighbouring separating elements 20 could have the function of lateral flow path defining elements.

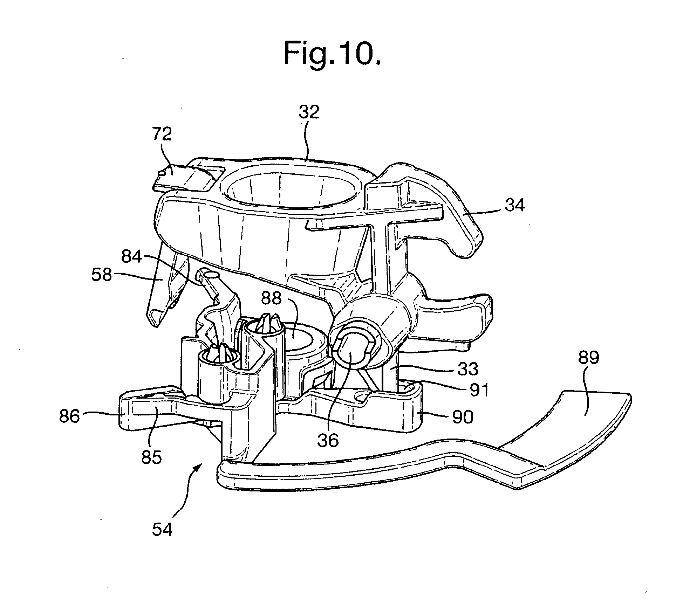

Each separating element 20 has a cavity-covering portion 28 which is in register with a respective cavity 16 in the base. Additionally, each separating element 20 has a centrally projecting portion 30, extending inwardly towards the centre of the disc assembly. An opening mechanism comprising an actuator 32 for lifting the separating elements 20 is provided. The actuator is in the form of a pivotable lever of polyoxymethylene (POM, e.g. Hostaform MT12U01) provided with jaws 34 for gripping the centrally projecting portions 30 of the separating elements 20. The actuator 32 has an energized position in which the jaws 34 are in a lowered position and an unloaded position in which the jaws 34 are in a raised position. The actuator 32 does not rotate with the disc assembly but remains oriented towards the mouthpiece; it is pivotable around the horizontal hinge 36 (see Figure 10) to move between raised (fired) and lowered (energised) positions.

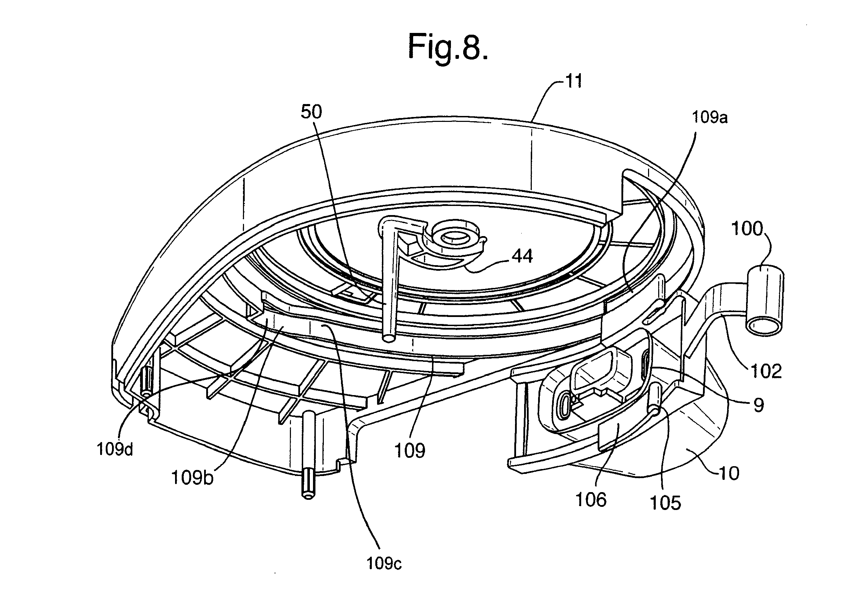

Referring to Figure 8, the upper outer cover component comprises, on its interior surface, a central cam 44, an elongate force transmitting member 50 and a cam track 109. The function of these components is explained below.

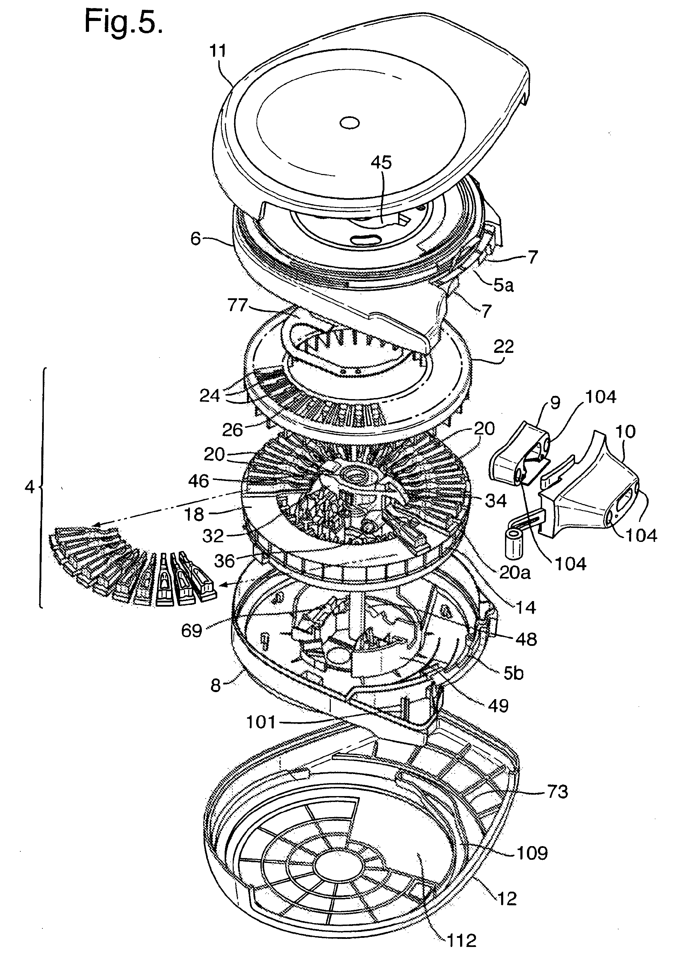

The inhaler housing (together with the mouthpiece assembly) are arranged to pivot with respect to the cover 11, 12 between a closed position in which the mouthpiece 10 is enclosed in the cover and an open position in which the mouthpiece is exposed for use. The central cam 44 engages with the actuator 32 to reset it when the inhaler is closed by rotating the main housing 6, 8 with respect to the outer cover 11, 12. As the cam 44 comes into contact with the jaws 34 of the actuator 32, the actuator 32 will rotate around its pivot 36. The jaws 34 will drop down to the primed or energized position of the actuator 32. The lowering of the jaws 34 will be against the force of a coil spring 46 of stainless steel which is biased to raise the jaws 34 to the unloaded position (although in fact the jaws are not totally unloaded in the raised position). The coil spring 46 is wound around a post 48 projecting upwardly from the lower housing portion 8 and may be seen, for example, in Figures 5, 6 & 7.

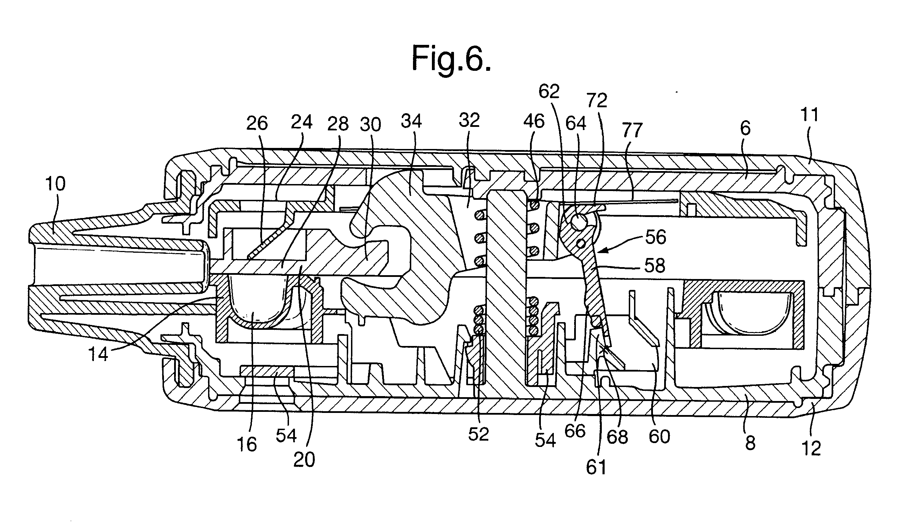

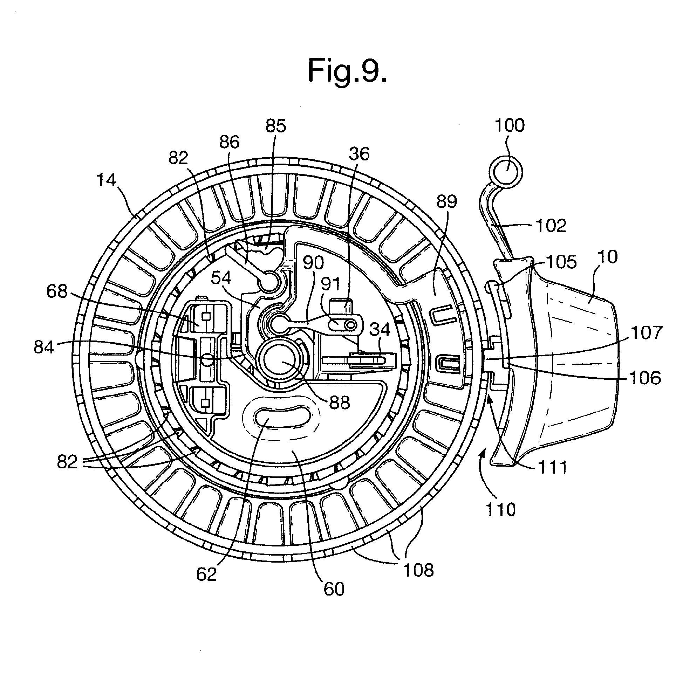

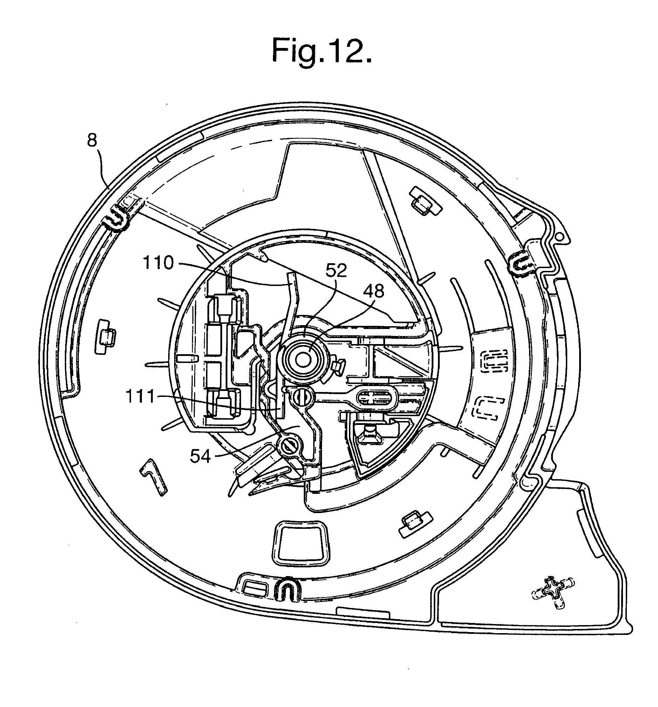

The force transmitting member 50 engages one end 110 of a torsion spring 52 of stainless steel located under the coil spring 46 and around the same post 48 (see Figures 6, 7 and 12). The torsion spring 52 is connected at its other end 111 to a drive member 54 of polyoxymethylene (POM, e.g. Hostaform MT12U01) for rotatingly advancing the cavities 16 by one increment at a time, so as each time to bring an unopened cavity into alignment with the mouthpiece 10. The drive member is best seen in Figures 9 and 12.

A latch 56 is provided to keep the actuator in the energized position. The latch 56 comprises a first element in the form of a prop 58 of polycarbonate (e.g. Makrolon 2458) and a second element in the form of a flap 60 of polyoxymethylene (POM, e.g. Hostaform MT12U01). The prop 58 has a first end portion 62 which is pivotable around a first horizontal pivot 64 on the actuator 32, near the opposite end of the actuator 32 to the jaws 34. The prop 58 has a second end portion 66 adapted to be supported on a shoulder 61 at one end of the flap 60. The flap 60 is pivotable around a second horizontal pivot 68 shown in Figure 6 simply by a cross indicating the axis of the pivot; the structure of the second pivot 68 is partly shown in Figure 5, where part of the support 69 for a corresponding pivot pin on the flap 60 is shown.

The flap 60 covers a flap valve aperture 70 provided in the lower housing portion 8, which may be seen in Figure 4. Air is allowed to enter the inhaler 2 through the aperture 70 when the user inhales through the mouthpiece 10 (outlet). The incoming air moves the flap, which in turn triggers the actuator 32 to open a cavity so that medicament may be entrained in the air flow. This will be explained in more detail below.

When the inhaler is in the open configuration shown in Figs 1, 3 and 4, air may enter the outer casing through an external air inlet71 between the outer casing and mouthpiece. A first part of an inlet air flow path is thus defined between the the outer cover and a region 113 of the side wall of the housing (see Figure 4).

From the first part of the inlet air flow path, air then passes between the flat internal face of the lower cover 12 and flat external surface of the lower housing 8 to reach the flap valve aperture 70 defined in the lower housing portion 8 (see Figure 4). This second part of the inlet air flow path is defined partly by a portion 112 of the inner surface of the lower outer casing component 12 (see Figure 5) which is kept free from reinforcing ribs which could impede or obstruct flow. The second part of the inlet air flow path is also partly defined by a slightly recessed region 114 of the lower housing 8 leading to the flap valve aperture 70. Figure 4 shows the underside of the lower housing portion 8 which has a number of apertures formed in it in addition to the flap valve aperture 70. One of these apertures is a dose counter window 117 through which a dose count number printed on the lower foil layer of the cavity disc becomes visible when the inhaler is in a closed configuration. In the closed configuration the dose counter window 117 is in registry with a dual purpose display window 119 in the lower casing member 12, such that the dose count is visible to a user. This is best understood with reference to Figure 13. Figure 2 shows the inhaler from underneath in the closed configuration, with the dose count number displayed in the dual purpose window 119.

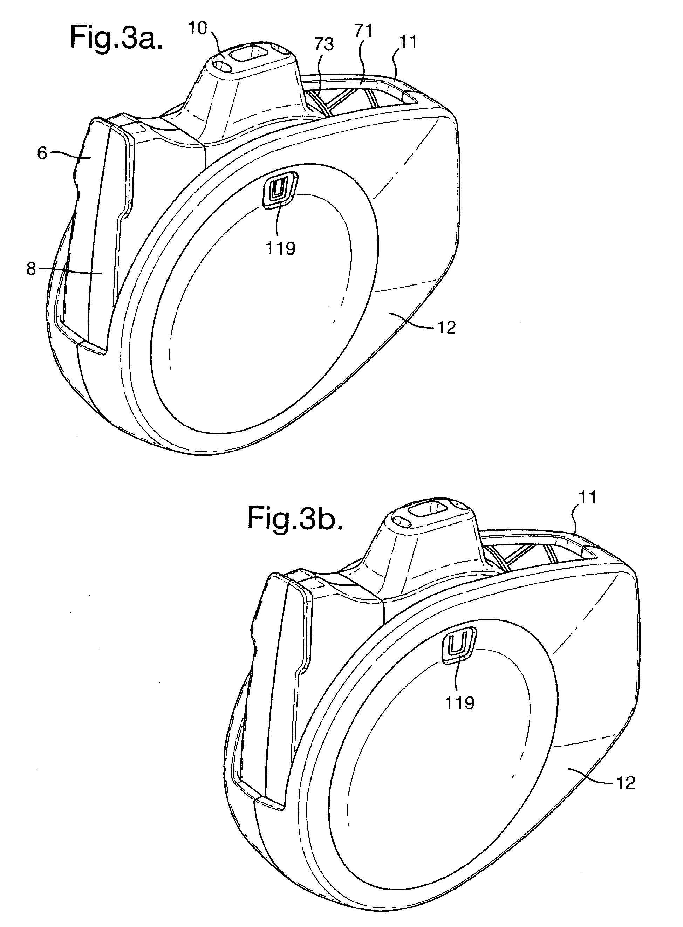

Referring again to Figures 4 and 13, a "ready for use" indicator window 118 is also provided in the underside of the lower housing portion, though which a display flag component 89 on the drive member 54 may be viewed. The ready for use indicator window comes into registry with the dual purpose display window 119 when the inhaler is in an open configuration, allowing the flag 89 to be viewed instead of the dose count number. The flag 89 has two indicia on it, one indicating that the inhaler is ready for use, and the other indicating that the current medicament cavity has been emptied. The position of the display flag changes in dependence on the state of the inhaler, so that the appropriate indicium is visible through the windows 118, 119. The structure and operation of the drive member 54 and display flag 89 will be discussed more fully below, but the states of the "ready for use" indicator may be understood with reference to Figures 3a and 3b which show, respectively, the "ready" and "fired" states of the inhaler.

When the inhaler is in the closed configuration (Figure 2), the mouthpiece assembly 9, 10 is received into the external air inlet 71, closing off the inlet and thereby helping to protect both the mouthpiece and the inlet air flow paths from being contaminated by particles of dirt.

Referring again to Figure 4, a number of apertures 116 are shown in addition to the dose counter and ready for use windows 117, 118. These further apertures 116 are a result of the injection moulding process and are necessary in order for various parts of the inhaler to be moulded in one piece with the lower housing. A transparent membrane 115 is shown separated from the lower housing portion 8. In the assembled inhaler the transparent membrane 115 is secured by adhesive to the lower surface of the lower housing 8 thereby sealing the moulding apertures 116 as well as the windows 117,118 and helping to prevent leakage air flow paths. It is important to minimise leakage air flow paths because they can reduce the air flow in the main air channels of the inhaler when the inhaler is used to values below those needed for correct functioning. They can also cause unpredictability in the air flow.

Because the membrane 115 is transparent, it allows the dose count and ready for use indicia to be viewed through the respective windows 117,118. It is possible to prepare the membrane 115 as a self adhesive film of polymer material, which is simple to assemble to the housing. The entire membrane may be coated with transparent adhesive or, alternatively, the portions of the membrane corresponding to the windows 117,118 may be left free of adhesive.

Fig. 6 is a schematic cross-sectional view of selected details of the inhaler, showing the inhaler in a primed state with the actuator 32 latched in an energized position. The jaws 34 of the actuator 32 have been lowered against the force of the coil spring 46. The dispensing assembly 4 is rotated, after the jaws 34 are lowered, to bring the next unopened cavity 16 into alignment with the mouthpiece 10. The jaws 34 now enclose the centrally projecting portion 30 of a separating element 20 associated with the unopened cavity. The second end portion 66 of the prop 58 is supported by the shoulder 61 of the flap 60. The latch 56 comprising the prop 58 and the flap 60 is now in its first position, in which it latches the actuator 32 in the energized position. The prop 58 is biased into the position shown in Figure 6 by the actuator, under the influence of the coil spring 46. The interface or contact point between the second end portion 66 of the prop 58 and the flap shoulder 61 is located such that the line of action of the force exerted by the prop on the shoulder is on the same side of the second horizontal pivot 68 (the flap pivot) as the portion of the flap 60 covering the aperture 70. In this way, the flap 60 is held in the illustrated lowered position. As long as the flap 60 remains still, the prop 58 is also prevented from moving, keeping the actuator 32 latched in its energized position.

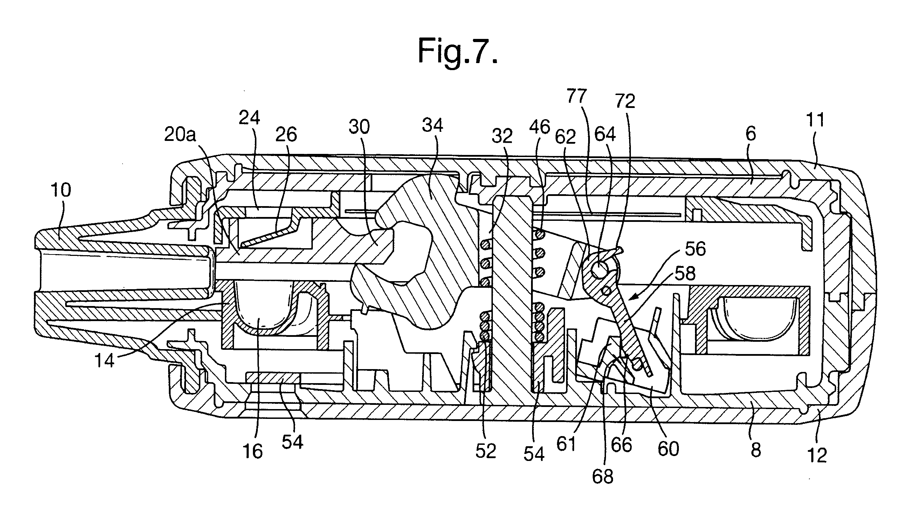

In order to administer a dose, the user inhales, creating a sufficient pressure drop across the flap 60 to raise the flap against the biasing force. This is illustrated in Fig. 7. As the flap 60 is raised and pivoted around the second pivot 68 (clockwise in Fig. 7), the flap shoulder 61 moves to the right in Figures 6 and 7 which results in the prop rolling off the shoulder 61 under the influence of the actuator spring (coil spring 46), the prop pivoting at its upper end around the pivot 64.

During the critical part of the movement, while the prop is being moved by the breath flap against a biasing force, the contact between the prop and shoulder is a rolling contact; the prop and breath flap behave as an over-centre mechanism. This is to minimise friction, which may add to the force required to trigger the mechanism and, more importantly, potentially cause this force to be unpredictable. During this rolling phase of the movement, the line of action of the force exerted on the flap shoulder by the prop moves from the left side of the flap pivot to the right side of the flap pivot. Once this has happened, the flap is no longer biased into the closed position by the prop; pivoting of the flap now continues rapidly, assisted by the force exerted by the prop under the influence of the coil spring 46.

The shape of the shoulder 61 is such that, when the flap reaches a certain angle, the prop will be pushed completely off the shoulder under the influence of the coil spring 46. This last stage of the movement will involve sliding friction, but the movement is driven entirely by the coil spring 46 and is independent of any movement of the breath flap; the coil spring is designed easily to overcome any friction between the prop and the shoulder.

The latch 56 is now in its second position, in which the actuator 32 is free to move to its unloaded position under the influence of the coil spring 46. The actuator 32 will rotate around its pivot 36 and the jaws 34 will be raised. The engaged separating element 20 which is in registry with the jaws 34 is thereby lifted from the cavity disc 14. The portion of the foil layer 18 associated with the cavity 16 which has been opened remains attached to the separating element 20. Figs. 5 and 7 illustrate one separating element 20a in the raised position being raised by the jaws 34 of the actuator 32. On inhalation, air flows across the top of the opened cavity inducing a circulating flow in the cavity which deaggregates medicament powder (not shown) in the cavity and entrains it in a flow of air exiting the inhaler through the mouthpiece 10. Details of the process of emptying the cavity may be found in co-pending applications numbers PCT/SE2008/051488 (WO 2009/082341) and US 61/222209 (from which WO 2011/002406 claims priority).

With the prop 58 in the un-latched position shown in Figure 7, the flap 60 is free to return to the lowered position after a dose is dispensed, however the actuator 32 remains in the unloaded position (Fig. 7) until the user primes the inhaler for the next dose.

Closing of the inhaler after use involves rotating the upper and lower casing components 11, 12 (the outer cover) with respect to the rest of the device to achieve the configuration shown in Figure 2. As this is done, the central cam feature 44 (see Figure 8) on the upper outer casing 11 engages with the actuator 32 to lower it and energise the coil spring 46. The central cam 44 accesses the actuator 32 via an aperture 45 in the upper housing portion 6, best seen in Figure 5. In the closed position, the actuator 32 is retained by the central cam 44 so that there is no possibility of a medicament cavity being opened whilst the inhaler is in the closed configuration.

The flap 60 has a protrusion, or flap cam 62 (see Figure 9), on its upper surface which engages with the force transmitting member 50 (see Figure 8) which depends from the upper casing component 12. The inter-engagement of these features retains the breath flap in the lowered position. The force transmitting member 50 and flap cam 62 remain engaged as the inhaler is opened, until the fully open configuration is reached, or nearly reached, at which point the flap 60 is released. This arrangement is provided to reduce the possibility of the flap being deflected by an inhalation when the inhaler is only partly open, which may result in incorrect functioning of one or more of the other components in the inhaler.

As the inhaler is closed, an indexing mechanism moves the cavity disc around to position an unopened cavity adjacent the mouthpiece 10 and the actuator 32.

The indexing mechanism comprises a drive member or indexer 54 including an integral pawl 85, an additional pawl 86, a display flag 89 and a pulling arm 90 (also known as an indexer link), as well as the torsion spring 52 and the mouthpiece 10.

Figure 9 shows the cavity disc 14 and indexing mechanism from below. The indexer or drive member 54 is shown in detail in Figure 10 together with the additional pawl 86, display flag 89, indexer link 90, actuator 32 and prop 58. The drive member 54 is a single moulding of polyoxymethylene (POM, e.g. Hostaform MT12U01) which comprises an integral pawl 85, prop preventer arm or catch 84, display flag 89 and mounting aperture 88. The drive member 54 is mounted via its mounting aperture 88 to the central post 48 on the lower housing 8 (the housing and post are not shown in Figure 9 but may be seen e.g. in Figure 5). The additional pawl 86 is a separate moulding (e.g. a POM moulding), attached to the indexer 54 by a snap fit pivot connection. Both the integral pawl and additional pawl engage with or between teeth 82 on the internal circumference of the cavity disc 14. The indexer link 90 is also a separate moulding of polypropylene (or alternatively a POM moulding), attached to the indexer 54 by a snap fit pivot connection. The indexer link includes a hole 91 into which projects a peg 33 on the lower side of the actuator 32 (see Figure 10). The functioning of the indexing mechanism will be described more fully below.

The mouthpiece assembly comprises a mouthpiece member 10 and an elastomeric seal member 9. The mouthpiece member 10 is a single moulding from polypropylene (e.g. Purell HM671T) which comprises a main part and a pivot ring 100 on the end of an arm 102 extending from the main part (see Figure 11). The mouthpiece assembly is pivotally mounted on a spigot 101 on the lower housing portion 8 (see Figure 5) which passes through the pivot ring 100. The main part of the mouthpiece assembly, best seen in Figure 11, comprises an inhalation channel 103 and secondary bypass channels 104 on each side. The secondary bypass channels 104 are partly formed by the mouthpiece member 10 and partly by the seal member 9.

On the side which faces the inhaler housing 6, 8, a leaf spring 106 projects obliquely from each of the upper and lower edges. At the distal end of each leaf spring 106 is a cam follower peg 105. The cam followers 105 and leaf springs 106 are integrally moulded with the mouthpiece member 10. The cam followers 105 and leaf springs 106 engage in cam tracks 109 on the inside surfaces of the respective outer covers / casing components 11, 12. The cam track 109 on the lower casing component can be seen in Figure 5, whilst the cam track 109 on the upper casing component 11 can be seen in Figure 8.

Projecting towards the inhaler housing 6, 8 from underneath the inhalation channel 103 of the mouthpiece member 10 is a spacing member or locating peg 107. On the outside circumference of the cavity disc 14 are notches 108 (see e.g. Figure 9) into which the locating peg 107 can project. In this way, the mouthpiece acts as a brake on the cavity disc to prevent indexing of the disc occurring at the wrong time in the indexing sequence.

During the opening and closing of the inhaler, the cam followers 105 and leaf springs 106 travel along the tracks 109 which control the movement of the mouthpiece towards and away from the cavity disc 14, and hence the engagement and disengagement of the locating peg 107 with the notches 108 of the cavity disc. In Figure 8, the mouthpiece and upper casing component 11 are shown when the inhaler is open; the leaf spring and cam follower are in region 109a of the cam track which brings the mouthpiece towards the housing and cavity disc (not shown) such that the disc is braked. A region 109b at the other end of the cam track 109 can be seen in Figure 8; in the region 109b, the track is further away from the disc assembly and comprises a widened portion 109c and a narrower terminal region 109d. When the spring and follower 106, 105 are moved into this region of the track, in the final stages of closing the inhaler, they enter the widened portion 109c, which leads the cam/spring radially outwardly with respect to the disc, and then finally the proximal end of the leaf spring 106 engages in the narrow terminal portion 109d of the track. The mouthpiece is thereby moved away from the cavity disc and housing and then retained securely in that position, releasing the disc to index. This will be explained more fully below.

The resilience of the leaf spring 106 means that the spacing member or locating peg 107 is resiliently brought to bear against the disc 14 when in the braking position. This allows tolerance in the disc mounting to be taken up. The inner edge of the disc 14 (which should more correctly be called an annulus rather than a disc) bears on a bearing flange 49 (see Figure 5) which is an integral part of the lower housing moulding 8. When the brake is engaged, the inner edge of the disc 14 will be biased against the bearing flange 49 in the region of the mouthpiece.

In this state, a precisely defined spacing 110 exists between the disc assembly and the mouthpiece assembly, through which air may pass on inhalation into the secondary bypass channels 104. Air may also pass through a smaller spacing 111 between the inlet of the inhalation channel 103 and the edge of the disc assembly. These spacings are best seen in Figure 9. It is desirable for the dimensions of these spacing to be as well-defined as possible so that the flow patterns and flow resistance are as well-defined as possible. This is achieved by the spacing member 107 being biased into engagement with the disc 14.

The smaller spacing 111 forms an annular bypass channel around the inhalation channel inlet, which may create a "sheath" flow of air around the main flow of particle-laden air from the disc cavity. Since the bypass air does not have particles of powder entrained in it, it may be able to form a barrier between the drug particles and the wall of the inhalation channel, reducing the deposition of drug particles on the wall.

The wall of the upper and lower housing portions 6,8 have cutaways 5a, 5b respectively which together form an aperture when the housing is assembled through which air passes into the mouthpiece. The mouthpiece seal member 9 forms a seal against the housing wall around this aperture. Baffles 7 are provided on each side of the cutaway 5a in the upper housing portion 6. The function of these baffles is to extend across the front of the cavities on each side of the cavity which is aligned with the mouthpiece inhalation channel 103. This helps to prevent any stray powder from a used cavity from being entrained in the bypass flow through the secondary bypass channels 104; air entering the bypass channels may do so from underneath the baffles, via the cutaway 5b in the lower housing portion.

After a dose has been dispensed, the user closes the inhaler. Through the rotation of the outer cover or casing components 11, 12 relative to the housing 6, 8, the central cam 44 will urge the actuator 32 to move to its energized position. Thus, the jaws 34 of the actuator 32 will move from the raised unloaded position illustrated in Figure 7 to the lowered energized position illustrated in Figure 6.

Substantially simultaneously with the cam 44 urging the actuator 32 into the energised position, the projecting second force transmitting member 50 on the upper outer casing 12 will urge the indexing mechanism to advance the next cavity 16 to be aligned with the mouthpiece 10. More particularly, the projecting member 50 (see Figure 8), passing through the aperture 45 in the upper housing portion (see Figure 5) engages with the torsion spring 52 to energise it. Figure 12 shows the torsion spring 52 mounted on the central post 48 of the lower housing portion 8. The spring 52 is at rest as shown in Figure 12. A first end 110 of the spring 52 is moved clockwise (as viewed in Figure 12) by the force transmitting member 50. A second end 111 of the spring is engaged with the drive member 54. The energized torsion spring 52 will thus urge the connected drive member 54 to rotate around the central axis provided by the post 48 in order to engage the cavity disc 14 and to thereby cause the disc 14 to rotate so as to bring the next cavity 16 into alignment with the mouthpiece 10. However, the force on the drive member 54 provided by the projecting member 50 via the torsion spring 52 is temporarily counteracted, at least until the actuator 32 has reached its energized position, by the mouthpiece brake arrangement described above. The indexing of the disc is thereby prevented until just before the inhaler is closed (when the leaf springs 106 and cam followers 105 reach region 109b in the cam tracks). This arrangement prevents the mechanism trying to index before the actuator is lowered (in which case the actuator would obstruct indexing). It also avoids the possibility of partial indexing if the cover is partially closed and then opened.

As illustrated in Fig. 9, before the brake is released the pawl 85 of the drive member 54 engages one of a plurality of teeth 82 in the disc 14. The prop preventer 84 is in a preventing position, engaged with the prop 58 to prevent it resting on the flap shoulder 61. Thus, in this state of the inhaler, the actuator cannot become latched in the energized position. This reduces the risk of re-firing from the same cavity 16.

As the brake is released, the drive member 54 will move under the influence of the torsion spring 52 and rotate the disc 14 by one cavity. The additional pawl 86 referred to above prevents the drive member 54 from over-rotating the disc 14, ensuring that the inhaler is indexed only one cavity at a time.

At the upper end of the prop 58 is a position-keeping projection 72 which engages with a steel prop spring 77 (best seen in Figure 5) mounted on the inside face of the upper housing portion 6. The prop spring 77 biases the prop 58 laterally against the shoulder 61 of the flap 60. As the drive member 54 rotates the disc 14 the prop preventer 84 will be removed from the preventing position, thereby allowing the prop 58 to become supported by the flap shoulder 61 and latch the energized actuator. The inhaler is now primed.

As previously described, when the user opens the inhaler and inhales through the mouthpiece 10, the flap 60 is raised so that the prop 58 comes off the flap shoulder 61, thereby unlatching the actuator 32. The actuator 32 will be raised under the influence of the coil spring 46 so that the jaws 34 of the actuator 32 remove the separating element 20 and a portion of the foil layer 18 from the cavity 16 presently aligned with the mouthpiece 10. As can be seen in e.g. Fig. 9, a movable pulling arm or indexer link 90 connects the drive member 54 with the actuator 32. As the actuator 32 and the jaws 34 are raised from the primed to the fired state, the pulling arm / indexer link 90 is moved laterally, shifting the drive member 54 round on the post 48, such that the pawl 85 slips back over one ratchet tooth 82 on the disc. The prop preventer catch 84 will consequently be moved back to its preventing position, in which the prop 58 is prevented from engaging the flap shoulder 61. When the user then closes the inhaler, it will once again become primed, following the sequence described above.

If the user, for some reason, does not close the inhaler fully, the spring 106 and cam follower 105 travelling in the track 109 will not reach its point of release (the region 109b of the cam track 109), and consequently the mouthpiece brake 10 will not be released. This in turn means that there will be no indexing. Furthermore, although the actuator 32 is in its energized position, it will not become latched, as latching can only occur in connection with indexing, as explained above - until the indexer (drive member 54) moves round, the prop preventer 84, which is an integral part of the indexer, will prevent latching. If the user then opens the inhaler again after not fully closing it, the actuator 32 will simply move back to its unloaded position.

The sequence of events on opening and closing the inhaler is set out in Table 1 below.

**Table 1**

| | | |
|---|---|---|
| 1 | Closed state - ready to use | Mouthpiece assembly 9,10 is enclosed by cover; external air inlet 71 is blocked by the housing. |
| | | Actuator 32 is energised, but held in lower position by central cam 44 on upper outer cover 11 |
| | | Prop 58 is spring biased into position to support actuator 32 but actuator is not held in energised state by prop at this stage. Prop preventer 84 on drive member / indexer 54 is disengaged with prop. |
| | | Brake (mouthpiece assembly) is disengaged from edge of cavity disc. |
| | | Torsion spring 52 is partly energised, biasing disc 14 into correct position. An unused cavity 16 in the disc is aligned with the mouthpiece |
| 2 | Open device | Central cam 44 disengages from actuator 32; actuator now held in energised state by prop 58 resting on flap shoulder 61. |
| | | Mouthpiece is exposed and external air inlet 71 adjacent mouthpiece is opened. |
| | | Brake (mouthpiece) is applied to prevent disc from rotating |
| | | Indexer spring 52 is relaxed as the device is opened |
| 3 | Inhale | Flap 60 moves, dislodging prop 58; actuator 32 is triggered and cavity lid / separating element 20 is lifted |
| | | Actuator 32 pulls indexer link (pulling arm) 90 and the pawl 86 of the indexer / drive member 54 is ratcheted around the teeth 82 on the cavity disc. Indexer spring 52 is still relaxed. |
| 4 | Start to close device | Actuator32 is re-set against force of coil spring 46 by cam 44 on upper outer casing moving relative to actuator, closing lid (separating element 20) on emptied cavity. |
| | | Indexer spring 52 is re-set to energised state by force transmitting member 50 on upper outer casing 11. Brake still applied; disc does not move |
| | | Prop preventer 84 engages flap 60 and stops actuator32 from latching (to prevent possibility of latching the mechanism before indexing). |
| 5 | Finish closing device | Actuator32 remains in energised state - no change |
| | | Mouthpiece is enclosed by cover and external air inlet is blocked by housing. |
| | | Brake is released, allowing disc to be advanced by the indexer under the influence of the indexer spring. Prop preventer disengages from flap 60 to allow prop 58 to be moved against flap 60 under influence of prop spring 77. Prop 58 is in position to take load of spring-biased actuator when cam 44 disengages on opening. |

It should be noted that in this application terms such as "upper", "lower", "above", "below" have been used for explanatory purposes to describe the internal relationship between elements of the inhaler, regardless of how the inhaler is oriented in the surrounding environment. For instance, in the exemplified embodiment in the drawings, the cavities 16 are regarded as being placed "below" the foil layer 18, while the separating elements 20 are regarded as being placed "above" the foil layer 18, regardless of how the inhaler 2 as a whole is held or turned by the user. Similarly, "horizontal" means a direction located in the plane of the foil layer 18 or any plane parallel to the plane of the foil layer 18, and "vertical" means any direction perpendicular to such planes. Thus, a vertical line may intersect the cavities 16, the foil layer 18 and the separating elements 20.

## Claims

1. An inhaler (2) comprising:
- a housing (6, 8) enclosing medicament and a dispensing system (4), the housing further including a mouthpiece (10);
- a cover (11, 12) movably mounted to the housing so as to be movable between an open configuration of the inhaler in which the mouthpiece is exposed for use and a closed configuration of the inhaler in which the mouthpiece is enclosed;
- wherein:
- in the open position, the cover (11, 12) at least partly defines an inlet aperture (71) to an inhalation air flow path leading through the inhaler and communicating with the mouthpiece and wherein, in the closed position, the mouthpiece is received into the inlet aperture (71), and
- the inhaler (2) has a generally flat shape with opposed major surfaces, **characterised in that** in the closed configuration the major surfaces are provided substantially entirely by the cover (11, 12).

2. An inhaler (2) as claimed in claim 1 wherein the inlet aperture (71) is defined between the housing (6, 8) and the cover (11, 12) when the cover is in the open configuration.

3. An inhaler (2) as claimed in claim 1 or claim 2 wherein, in the closed configuration, the inlet aperture (71) is closed.

4. An inhaler (2) as claimed in any preceding claim wherein, in the open configuration, the inlet aperture (71) is located adjacent the mouthpiece (10).

5. An inhaler (2) as claimed in any preceding claim, wherein in the open configuration the mouthpiece (10) projects from between the major surfaces.

6. An inhaler (2) as claimed in any preceding claim wherein the inlet aperture (71) is located in an edge of the cover (11, 12) between the major surfaces.

7. An inhaler (2) as claimed in any preceding claim wherein the housing (6, 8) has an overall disc shape, the mouthpiece (10) projecting from an edge of the disc.

8. An inhaler (2) as claimed in claim 7 wherein the housing (6, 8) has a handle portion projecting from the disc, whereby a user may move the housing with respect to the cover (11, 12) thereby changing between open and closed configurations of the inhaler.

9. An inhaler (2) as claimed in any preceding claim wherein the cover (11, 12) is pivotally mounted on the housing (6, 8).

10. An inhaler (2) as claimed in any preceding claim wherein at least one display aperture (117, 118) is defined in the housing (6, 8), through which one or more indicia within the housing are viewable.

11. An inhaler (2) as claimed in claim 10 wherein the cover (11, 12) defines at least one display window (119) which in one or both of the open and closed configurations is in registry with at least one said display aperture (117, 118) whereby at least one of said indicia is viewable.

12. An inhaler (2) as claimed in claim 11 wherein a plurality of the said indicia are provided, and wherein a single said display window (119) in the cover (11, 12) comes into registry with different ones of the said display apertures (117, 118) in the open and closed inhaler configurations, respectively, whereby different ones of the indicia become viewable in the open and closed inhaler configurations, respectively.

13. An inhaler (2) as claimed in any of claims 10 to 12 wherein the housing (6, 8) has an overall disc shape and a first said display aperture (117, 118) is defined in a major face of the said disc, a transparent membrane extending over the said first display aperture and being bonded to the said major face.

14. An inhaler (2) as claimed in claim 13, wherein the said major face of the disc has at least one further aperture defined in it, in addition to any display aperture(s), the said at least one further aperture (116) being covered by the said membrane.

## Patentansprüche

1. Inhalator (2), umfassend:
- ein Gehäuse (6, 8), das ein Medikament einschließt, und ein Ausgabesystem (4), wobei das Gehäuse ferner ein Mundstück (10) aufweist;
- eine Abdeckung (11, 12), die beweglich an dem Gehäuse befestigt ist, um zwischen einer offenen Konfiguration des Inhalators, in der das Mundstück für die Verwendung freigelegt ist, und einer geschlossenen Konfiguration des Inhalators, in der das Mundstück eingeschlossen ist, bewegt werden kann;
- wobei:
- in der offenen Position die Abdeckung (11, 12) mindestens teilweise eine Einlassöffnung (71) zu einem Inhalierluftströmungsweg, der durch den Inhalator führt, definiert und mit dem Mundstück kommuniziert, und wobei in der geschlossenen Position das Mundstück in der Einlassöffnung (71) aufgenommen ist, und
- der Inhalator (2) eine im Allgemeinen flache Form mit gegenüberliegenden Hauptoberflächen aufweist, **dadurch gekennzeichnet, dass** in der geschlossenen Konfiguration die Hauptoberflächen im Wesentlichen vollständig von der Abdeckung (11, 12) bereitgestellt werden.

2. Inhalator (2) nach Anspruch 1, wobei die Einlassöffnung (71) zwischen dem Gehäuse (6, 8) und der Abdeckung (11, 12) definiert ist, wenn sich die Abdeckung in der offenen Position befindet.

3. Inhalator (2) nach Anspruch 1 oder Anspruch 2, wobei in der geschlossenen Konfiguration die Einlassöffnung (71) geschlossen ist.

4. Inhalator (2) nach einem der vorhergehenden Ansprüche, wobei in der offenen Konfiguration die Einlassöffnung (71) benachbart zu dem Mundstück (10) angeordnet ist.

5. Inhalator (2) nach einem der vorhergehenden Ansprüche, wobei in der offenen Konfiguration das Mundstück (10) zwischen den Hauptoberflächen hervorsteht.

6. Inhalator (2) nach einem der vorhergehenden Ansprüche, wobei die Einlassöffnung (71) in einem Rand der Abdeckung (11, 12) zwischen den Hauptoberflächen angeordnet ist.

7. Inhalator (2) nach einem der vorhergehenden Ansprüche, wobei das Gehäuse (6, 8) insgesamt eine Scheibenform aufweist, wobei das Mundstück (10) von einem Rand der Scheibe hervorsteht.

8. Inhalator (2) nach Anspruch 7, wobei das Gehäuse (6, 8) einen Griffabschnitt aufweist, der von der Scheibe hervorsteht, wodurch ein Benutzer das Gehäuse in Bezug auf die Abdeckung (11, 12) bewegen kann und zwischen offenen und geschlossenen Konfigurationen des Inhalators wechselt.

9. Inhalator (2) nach einem der vorhergehenden Ansprüche, wobei die Abdeckung (11, 12) schwenkbar an dem Gehäuse (6, 8) befestigt ist.

10. Inhalator (2) nach einem der vorhergehenden Ansprüche, wobei mindestens eine Anzeigeöffnung (117, 118) in dem Gehäuse (6, 8) definiert ist, durch die eine oder mehrere Markierungen in dem Gehäuse sichtbar sind.

11. Inhalator (2) nach Anspruch 10, wobei die Abdeckung (11, 12) mindestens ein Anzeigefenster (119) definiert, das in einer oder in beiden der offenen und geschlossenen Konfigurationen mit mindestens einer Anzeigeöffnung (117 , 118) ausgerichtet ist, wodurch mindestens eine der Markierungen sichtbar ist.

12. Inhalator (2) nach Anspruch 11, wobei mehrere der Markierungen bereitgestellt sind und wobei ein einzelnes Anzeigefenster (119) in der Abdeckung (11, 12) mit unterschiedlichen Anzeigeöffnungen (117, 118) in den offenen bzw. geschlossenen Inhalator-Konfigurationen ausgerichtet wird, wodurch unterschiedliche Markierungen in der offenen bzw. geschlossenen Inhalator-Konfiguration sichtbar werden.

13. Inhalator (2) nach einem der Ansprüche 10 bis 12, wobei das Gehäuse (6, 8) insgesamt eine Scheibenform aufweist und eine erste Anzeigeöffnung (117, 118) in einer Hauptseite der Scheibe definiert ist, wobei sich eine transparente Membran über die erste Anzeigeöffnung erstreckt und mit der Hauptseite verbunden ist.

14. Inhalator (2) nach Anspruch 13, wobei die Hauptseite der Scheibe mindestens eine weitere Öffnung darin zusätzlich zu jeder beliebigen Anzahl von Anzeigeöffnung(en) aufweist, wobei die mindestens eine weitere Öffnung (116) von der Membran bedeckt wird.

## Revendications

1. Inhalateur (2), comprenant:
- un boîtier (6, 8) contenant un médicament et un système de distribution (4), le boîtier comportant en outre un embout buccal (10);
- une coiffe (11, 12) qui est montée de façon mobile sur le boîtier de manière à être mobile entre une configuration ouverte de l'inhalateur, dans laquelle l'embout buccal est exposé en vue d'une utilisation et une configuration fermée de l'inhalateur, dans laquelle l'embout buccal est rentré,
dans lequel:
- dans la position ouverte, la coiffe (11, 12) définit au moins partiellement une ouverture d'entrée (71) vers un chemin d'écoulement d'air d'inhalation qui passe à travers l'inhalateur et qui communique avec l'embout buccal, et dans lequel, dans la position fermée, l'embout buccal est reçu dans l'ouverture d'entrée (71), et
- l'inhalateur (2) présente une forme essentiellement plate avec des surfaces majeures opposées, **caractérisé en ce que**, dans la configuration fermée, les surfaces majeures sont formées sensiblement entièrement par la coiffe (11, 12).

2. Inhalateur (2) selon la revendication 1, dans lequel l'ouverture d'entrée (71) est définie entre le boîtier (6, 8) et la coiffe (11, 12) lorsque la coiffe se trouve dans la configuration ouverte.

3. Inhalateur (2) selon la revendication 1 ou la revendication 2, dans lequel, dans la configuration fermée, l'ouverture d'entrée (71) est fermée.

4. Inhalateur (2) selon l'une quelconque des revendications précédentes, dans lequel, dans la configuration ouverte, l'ouverture d'entrée (71) est située à proximité de l'embout buccal (10).

5. Inhalateur (2) selon l'une quelconque des revendications précédentes, dans lequel, dans la configuration ouverte, l'embout buccal (10) fait saillie entre les surfaces majeures.

6. Inhalateur (2) selon l'une quelconque des revendications précédentes, dans lequel l'ouverture d'entrée (71) est située dans un bord de la coiffe (11, 12) entre les surfaces majeures.

7. Inhalateur (2) selon l'une quelconque des revendications précédentes, dans lequel le boîtier (6, 8) présente une forme générale de disque, l'embout buccal (10) faisant saillie à partir d'un bord du disque.

8. Inhalateur (2) selon la revendication 7, dans lequel le boîtier (6, 8) comprend une partie de poignée qui fait saillie à partir du disque, moyennant quoi un utilisateur peut déplacer le boîtier par rapport à la coiffe (11, 12), commutant de ce fait entre les configurations ouverte et fermée de l'inhalateur.

9. Inhalateur (2) selon l'une quelconque des revendications précédentes, dans lequel la coiffe (11, 12) est montée de façon pivotante sur le boîtier (6, 8).

10. Inhalateur (2) selon l'une quelconque des revendications précédentes, dans lequel au moins une ouverture d'affichage (117, 118) est définie dans le boîtier (6, 8), à travers laquelle un ou plusieurs repère(s) à l'intérieur du boîtier est (sont) visible(s).

11. Inhalateur (2) selon la revendication 10, dans lequel la coiffe (11, 12) définit au moins une fenêtre d'affichage (119) qui, dans l'une ou chacune des deux configurations ouverte et fermée, est alignée avec au moins une desdites ouvertures d'affichage (117, 118), moyennant quoi au moins un desdits repères est visible.

12. Inhalateur (2) selon la revendication 11, dans lequel il est prévu une pluralité desdits repères, et dans lequel une seule fenêtre d'affichage (119) dans la coiffe (11, 12) est alignée avec différentes desdites ouvertures d'affichage (117, 118) dans les configurations ouverte et fermée de l'inhalateur, respectivement, moyennant quoi différents repères deviennent visibles dans les configurations ouverte et fermée de l'inhalateur, respectivement.

13. Inhalateur (2) selon l'une quelconque des revendications 10 à 12, dans lequel le boîtier (6, 8) présente une forme générale de disque, et une première desdites ouvertures d'affichage (117, 118) est définie dans une face majeure dudit disque, une membrane transparente s'étendant sur ladite première ouverture d'affichage et étant attachée à ladite face majeure.

14. Inhalateur (2) selon la revendication 13, dans lequel ladite face majeure du disque comporte au moins une ouverture supplémentaire qui est définie dans celui-ci, en plus de la ou des ouverture(s) d'affichage, ladite au moins une ouverture supplémentaire (116) étant couverte par ladite membrane.
